(19) Europäisches Patentamt
European Patent Office
Office européen des brevets

(11) Publication number: **0 438 856 A2**

# (12) EUROPEAN PATENT APPLICATION

(21) Application number: **90311855.2**

(51) Int. Cl.$^5$: **A61K 31/40**

(22) Date of filing: **30.10.90**

The title of the invention has been amended (Guidelines for Examination in the EPO, A-III, 7.3).

(30) Priority: **31.10.89 JP 284098/89**

(43) Date of publication of application: **31.07.91 Bulletin 91/31**

(84) Designated Contracting States: **DE FR GB IT**

(71) Applicant: **SHISEIDO COMPANY LIMITED**
**5-5 Ginza 7-chome**
**Chuo-ku Tokyo(JP)**

(72) Inventor: **Fukuda, Minoru**
**245-8 Tana**
**Sagamihara-shi, Kanagawa(JP)**
Inventor: **Yagi, Eiichiro**
**2-3-10-506, Susukino, Midori-ku**
**Yokohami-shi, Kanagawa(JP)**
Inventor: **Naganuma, Masako**
**1-17-13, Tamagawa Den-enchofu**
**Setagaya-ku, Tokyo(JP)**
Inventor: **Mori, Wataru**
**2-20-13, Sengoku, Bunkyo-ku**
**Tokyo(JP)**

(74) Representative: **Hale, Stephen Geoffrey et al**
**J.Y. & G.W. Johnson Furnival House 14/18**
**High Holborn**
**London WC1V 6DE(GB)**

## (54) Use of melatonin to protect the skin against the influence of UV-rays.

(57) A protective pharmacological composition against skin aging or skin cancer caused by UV rays contains melatonin as the active ingredient. It may be in the form of an oral preparation or parenteral preparation (i.e. injection) which is administered at the dose of melatonin for a human adult of 1 to 3000 mg/day, or in the form of an external treatment preparation containing 1 to 10% by weight of melatonin.

EP 0 438 856 A2

# UV RAYS AGING PROTECTIVE PHARMACOLOGICAL COMPOSITION

## BACKGROUND OF THE INVENTION

### 1. Field of the Invention

The present invention relates to a protective pharmacological composition against UV rays. More specifically, it relates to a pharmacological composition for preventing skin aging by UV rays, for example, the appearance of wrinkles, splotches, freckles, yellowing, and sagging, including skin cancer, and containing melatonin as the active ingredient.

### 2. Description of the Related Art

Currently, the incidence of skin cancer has increased due to the destruction of the ozone layer, and an aging of the skin by UV-rays has become a social problem, and accordingly, there is an urgent need to develop a drug for protecting the skin from aging due to UV-rays.

Melatonin is physiologically active amine derivative having a structure of N-acetyl-5-methoxytryptamine, and has been considered to primarily permit the generative function of an animal to be synchronized with the optical period. There have been reports on a suppressing of a proliferation of tumors by an admnistration of melatonin to experimental leukemia (Burswell, 1975), sarcoma (Lapin and Ebels, 1976), an mammary tumors (Anisimov, 1973; Karmali, 1978; Aubers, 1980), but there are also reports that tumors were worsened in mammary tumors (Hamilton, 1969) or lung cancer (Lapin and Ebels, 1976), and the action thereof has not been clarified so far.

Also, when contained in a skin cosmetic, melatonin has been known to have a skin activating effect and skin roughening amelioration effect (Japanese Unexamined Patent Publication (Kokai) No. 61-221104), but there has been no report on the protective effect thereof against skin aging, broadly including skin cancer, and further, no quantitative consensus has been obtained of the protective effect thereof while taking its side effects into consideration.

## SUMMARY OF THE INVENTION

Accordingly, the objects of the present invention are to eliminate the above-mentioned disadvantages of the prior art and to provide a protective pharmacological composition capable of effectively preventing skin aging by UV-rays, including skin cancer.

Other objects and advantages of the present invention will be apparent from the following description.

In accordance with the present invention, there is provided a protective pharmacological composition against skin aging by UV-rays comprising:

(i) an effective amount of melatonin; and

(ii) a carrier therefor.

The protective pharmacological composition according to the present invention can be in the form of an oral preparation or a parenteral preparation (e.g., injection) at a dose of melatonin for a human adult of 1 to 3000 mg/day or in the form of an external treatment preparation containing 1 to 10% by weight of melatonin.

## BRIEF DESCRIPTION OF THE DRAWINGS

The present invention will be better understood from the description set forth below with reference to the accompanying drawings, wherein:

Figure 1 illustrates the difference in colony formation ratio depending on the presence or absence of a melatonin administration when cell damage by UV-rays irradiation is effected; and

Fig. 2 illustrates the difference in change with a lapse of time of the number of tumors generated, depending on the presence or absence of a melatonin administration when UV-rays were irradiated onto hairless mice.

## DESCRIPTION OF THE PREFERRED EMBODIMENTS

The present inventors have found that a pharmacological composition containing a predetermined

amount of melatonin suppresses cancer or an aging of the skin generated by UV-rays, without worsening the systemic condition.

The structural formula of melatonin usable in the present invention as the active ingredient is represented by:

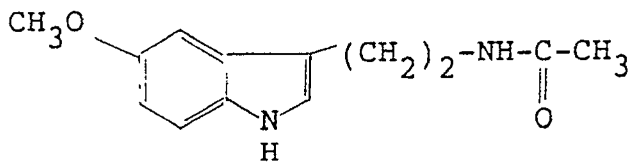

and can be prepared according to methods known in the art.

The protective pharmacological composition against skin aging by UV-rays, including skin cancer, can be prepared into various oral and parenteral dosage forms, such as tablets, pills, capsules, granules, liquids, injections, syrups and ointments, by mixing melatonin as the active ingredient with pharmaceutical carriers conventionally used, such as lactose, glucose, starch, sucrose, dextrin, celluloses, agar, magnesium stearate, aluminum silicate, talc, gelatin, lanolin, vegetable oil, petrolatum, gum arabic, polyalkylene glycol, tragacanth, fluid paraffin, and water.

If necessary, conventional preservatives, stabilizers, emulsifiers, and dissolution aids may be added.

The dose of the protective pharmacological composition against skin aging by UV-rays, including skin cancer, depends on the age of the patient, the condition of the disease, the body weight, age, and the administration method, the but the oral dose for a human adult is preferably 1 to 3000 mg/day, preferably 100 to 1000 mg/day as the amount of melatonin. If the dose is smaller than 1 mg/day, no effect is obtained as a protective drug against UV-rays aging, and if the dose is more than 3000 mg/day, denaturation may sometimes undesirably occur in the generative organs or liver. The administration is usually carried out 2 to 4 times per day. When administered intraperitoneally, intramuscularly or directly to the tumor site, it may be also used with the above-specified amount as the standard. In the case of external use (i.e., external treatment agent), it may be used in the form of 1 to 10% by weight, preferably 3 to 8% by weight, of a hydrophilic ointment or hydrophobic ointment.

The range of application for melatonin includes benignant tumors, apparent carcinoma, intraepidermal carcinoma, solar keratosis carcinoma, and infiltration carcinoma, which are cancers generated by UV-rays, and skin changes such as wrinkles, splotches, freckles, yellowing, and sagging.

As described above, the drug of the present invention has a protective effect against cell damage caused by UV-rays irradiation, and has a suppressive effect against UV-rays-generated carcinogenesis, and thus has a marked protective effect against skin aging by UV-rays, such as wrinkles, splotches, freckles, yellowing and sagging, including skin cancer.

## EXAMPLES

The present invention will now be further illustrated by, but is by no means limited to, the following Examples.

### Pharmacological tests

#### Test 1: Effect on cell damage caused by UV-rays irradiation

Fibroblasts derived from a human being were seeded in a laboratory dish having a diameter of 6 cm, at 400 cells/laboratory dish, and were cultured in Eagle's MEM medium containing 10% fetal calf serum in an incubator at 5% $CO_2$, at 37°C for 24 hours. After the medium was exchanged with a phosphate buffer containing 100 mM of melatonin (SIGMA Co.), it was irradiated by UVB (Toshiba FL-SE 20W) at 50 mJ/cm$^2$ or 100 mJ/cm$^2$, followed by a further culturing for 6 days. The culture was fixed with a 10% neutral formalin, stained with a 30-fold Gimza solution, and the colonies per laboratory dish were counted. The colonies of the irradiated group were divided by the colonies of the non-irradiated group, to measure the colony formation ratio. As a Control, a phosphate buffer containing no melatonin was added.

The results are shown in Fig. 1. In Fig. 1, (a) is the system containing no melatonin and (b) is the system containing 100 mM of melatonin. As can be seen from Fig. 1, both the group irradiated with 50 mJ/cm$^2$ of UV-ray and the group irradiated with 100 mJ/cm$^2$ of UV-ray to which melatonin was added (b)

show a significantly higher colony formation ratio ($p < 0.01$ as the result of t-examination). The risk ratio p becomes statistically significant at $p < 0.05$. Accordingly, it can be seen that melatonin has a protective effect against cell damage caused by UV-rays irradiation.

Test 2: UV-rays carcinogenesis suppression effect

Test animal:

Male albino hairless mice 8 weeks old (strain derived from Skh-HR-1, weight 25 - 35 g) were used, and one group consisted of 12 to 14 mice.

Sample and administration method:

Melatonin (SIGMA Co.) was used as a suspension in 20% ethanol containing physiological saline, and administered by an intraperitoneal injection of 0.1 ml in doses at 3 levels of 0 mg, 1 mg, and 10 mg per day.

Grouping:

The hairless mice were classified into 3 groups as shown in Table 1.

Table 1

| Grouping of Hairless Mice | | | |
|---|---|---|---|
| Group | Number | Sample administered | UV irradiation |
| A | 12 | Physiological saline containing 20% ethanol (solvent) | + |
| B | 13 | 1 mg melatonin | + |
| C | 14 | 10 mg melatonin | + |

Ultraviolet rays (UV) irradiation light source and irradiation schedule:

A Toshiba FL20-SE was mounted in a cage for irradiation, and irradiation was carried out at an irradiation intensity of 0.94 $mW/cm^2$, for 8 minutes per day, three times per week on alternate days for weeks. The irradiation was carried out 57 times, and the total irradiation dose was 25.7 $J/cm^2$. The administration of the sample was commenced simultaneously with the UV rays irradiation, and after the completion of the UV rays irradiation, the sample was administered continuously for an additional 10 weeks.

Observation item:

The size of the tumor generated was measured with slide calipers.

Data processing:

A t-examination was practiced for the number of tumors with sizes of 5 mm or more per one individual, and the number of tumors generated with sizes of 5 mm or more is shown in Fig. 2. In Fig. 2, o shows no melatonin-administered group (A), Δ the group administered with 1 mg of melatonin (B), and □ the group administered with 10 mg of melatonin (C). When the number of tumors generated with sizes of 5 mm or more was compared per one mouse, it was found that, in the melatonin administered group, the generation and proliferation of tumors was significantly suppressed ($p < 0.05$, on 23 days and 26 days and thereafter).

From the above, it can be seen that melatonin has a protective effect against skin cancer due to UV-rays. For the group administered with 10 mg of melatonin, although a protective effect against skin cancer was seen, denaturation occurred in the generative organs, and the liver became whitened.

Recipe examples

Formulation examples of the pharmacological composition of the present invention are shown as follows.

| Example 1 | |
|---|---|
| Melatonin | 5.0 - g |
| Lactose | 12.4 - g |
| Microcrystallaine cellulose | 12.4 - g |
| Corn starch | 0.1 - g |
| Magnesium stearate | 0.1 - g |

The above components were thoroughly mixed and 100 tablets prepared by the direct tabletting method. One tablet (300 mg) contained 50 mg of melatonin.

| Example 2 | |
|---|---|
| Melatonin | 10.0 - g |
| Lactose | 40.0 - g |

The above components were thoroughly mixed and 250 capsules prepared by a conventional method. One capsule contained 40 mg of melatonin.

| Example 3 | |
|---|---|
| Melatonin | 20.0 - g |
| Lactose | 79.1 - g |
| Magnesium stearate | 1.0 - g |

The above components were thoroughly mixed and 100 g of granules prepared by a dry system granulator. One gram of granule contained 200 mg of melatonin.

| Example 4 | |
|---|---|
| Melatonin | 5.0 - g |
| Polyethylene glycol 400 | 15.0 - g |
| Polyethylene glycol 2000 | 30.0 g |

The above components were thoroughly mixed to prepare 50 g of an ointment. One gram of ointment contained 100 mg of melatonin.

**Claims**

1. A protective pharmacological composition against skin aging by UV-rays comprising:
(i) an effective amount of melatonin; and
(ii) a carrier therefor.

2. A protective pharmacological composition as claimed in claim 1, in the form of an oral or parenteral preparation, which is administered at a dose of melatonin for a human adult of 1 to 3000 mg/day.

3. A protective pharmacological composition as claimed in claim 1, in the form of an external treatment preparation containing 1 to 10% by weight of melatonin.

4. The use of melatonin for the manufacture of a medicament for protection against skin aging by UV-rays.

5. The use of melatonin for the manufacture of a medicament for preventing generation of skin cancer by UV-rays.

# Fig.1

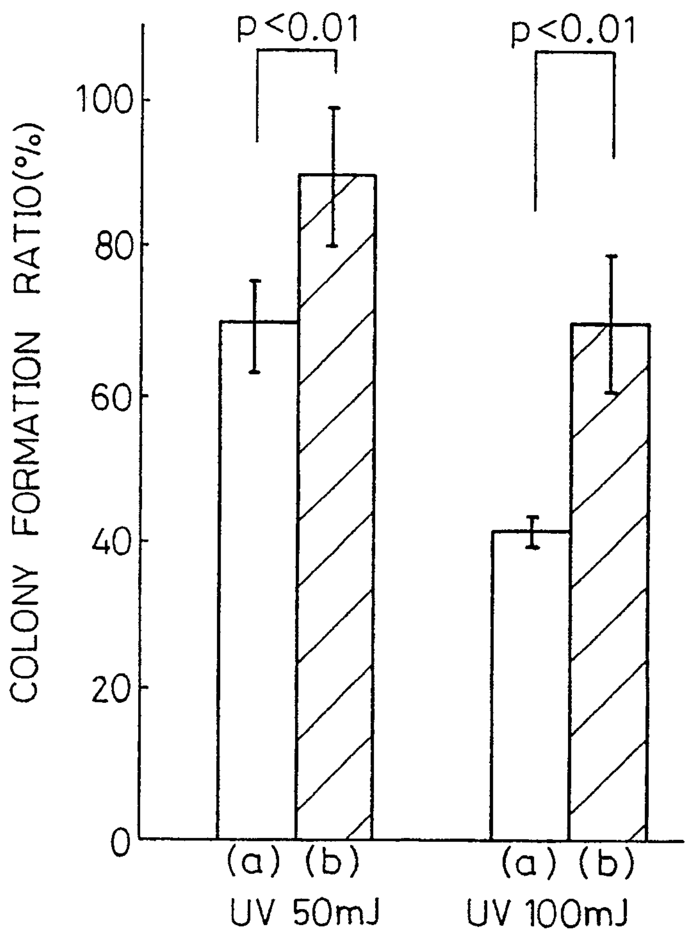
p<0.01
p<0.01
100
80
60
40
20
0
COLONY FORMATION RATIO(%)
(a) (b)
UV 50mJ
(a) (b)
UV 100mJ

Fig. 2

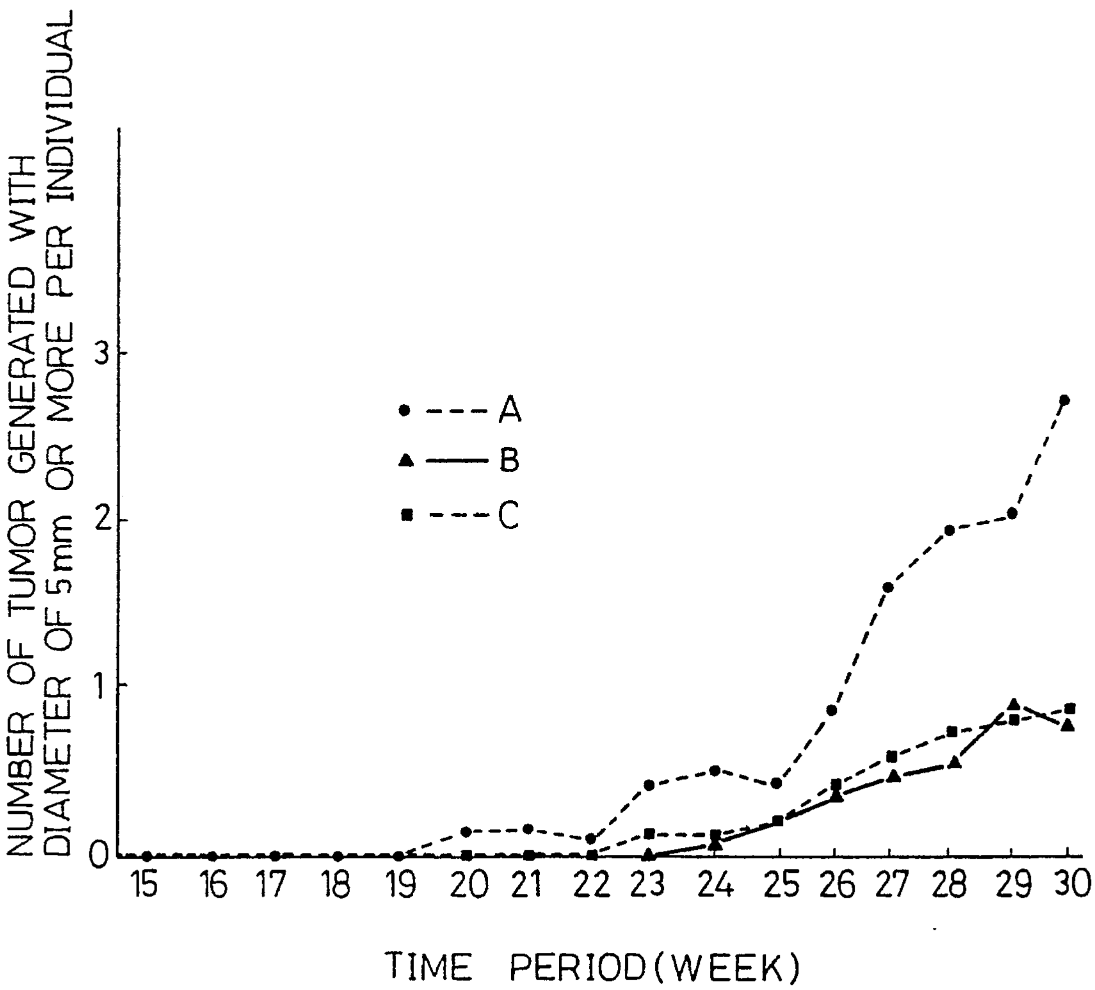
NUMBER OF TUMOR GENERATED WITH DIAMETER OF 5mm OR MORE PER INDIVIDUAL
3
2
1
0
A
B
C
15 16 17 18 19 20 21 22 23 24 25 26 27 28 29 30
TIME PERIOD (WEEK)